# EUROPEAN PATENT SPECIFICATION

(11) **EP 3 143 879 B1**
(45) Date of publication and mention of the grant of the patent: **22.01.2025**
(21) Application number: 15185867.7
(22) Date of filing: 18.09.2015
(51) Int. Cl.: A23C 9/127, C12N 9/64

(54) **YOGURT WITH REDUCED RIPENING**
JOGHURT MIT REDUZIERTER REIFUNG
YAOURT AVEC MATURITÉ RÉDUITE

(43) Date of publication of application: 22.03.2017
(73) Proprietor: DSM IP Assets B.V., 6221 BE Maastricht (NL)
(72) Inventor: Grabinski, Dominik Bohdan, 6100 AA ECHT (NL)
(74) Representative: dsm-firmenich IP

(56) References cited:
- WO-A1-2013/164481
- WO-A1-2015/128417
- US-A- 4 576 822
- US-A1- 2005 095 316
- US-A1- 2005 095 317
- US-A1- 2011 008 492
- US-A1- 2014 072 670
- LANGHOLM JENSEN J ET AL: "Camel and bovine chymosin: the relationship between their structures and cheese-making properties", ACTA CRYSTALLOGRAPHICA SECTION D: BIOLOGICAL CRYSTALLOGRAPHY, MUNKSGAARD PUBLISHERS LTD. COPENHAGEN, DK, vol. 69, no. Part 5, Sp. Iss. SI, 1 May 2013 (2013-05-01), pages 901 - 913, XP002717517, ISSN: 0907-4449, [retrieved on 20130419], DOI: 10.1107/S0907444913003260

## Description

### Field of the invention

The present invention relates to a process for the production of a fermented milk product, which is a yogurt. According to another aspect, the present invention relates to yogurt having a reduced ripening. According to yet another aspect the present invention relates to a kit of parts. According to still another aspect the present invention relates to use of a polypeptide having chymosin activity.

### Background of the invention

The food industry uses different bacteria, in the form in particular of ferments, in particular lactic acid bacteria, in order to improve the taste and the texture of foods but also to extend the shelf life of these foods. In the case of the dairy industry, lactic acid bacteria are used intensively in order to bring about the acidification of milk (by fermentation) but also in order to texturize the product into which they are incorporated. Among the lactic acid bacteria used in the food industry, there can be mentioned the genera *Streptococcus* and *Lactobacillus.* The lactic acid bacterial species *Streptococcus thermophilus* and *Lactobacillus delbrueckii* ssp *bulgaricus* are used in particular in the formulation of the ferments used for the production of fermented milks, for example yogurts.

The acidity produced in yogurt depends mainly on the acidifying activity of the yogurt culture (*Streptococcus thermophilus* and *Lactobacillus delbrueckii* ssp. *bulgaricus*) and therefore the amount of lactic acid produced during the milk maturation and also the residual acidity produced during cold storage. The texture is also varying during storage and participates in the final product sensorial properties. The recipe of the yogurt has also an impact on the yogurt sensorial properties by modifying the texture or the aroma perception.
Fermented milk products such as yogurts, are often fortified with extra protein in order to increase the thickness of the products. Proteins mostly used for this purpose are milk protein sources such as caseinates, whey protein isolates and skim milk powder.

Protein prices are increasing because of increasing demand. This is also true for milk proteins. Fortification of fermented milk products with milk proteins is thus becoming more expensive. As a result dairy companies are looking for opportunities to reduce the milk protein content that is used for fortification of the fermented milk products.

Reduction of milk protein content in fermented milk products comes at a cost. Milk proteins are key in generating a certain protein gel strength within the dairy product. Reduction of the protein content thus leads to reduction of the gel strength, and as a result the thickness of the yogurt in sensory perception is reduced. This is undesirable and puts a strong restriction on the extent with which the protein content can be reduced in fermented milk products. The solution for reduction of the protein content is to find a means to compensate for the loss in gel strength. There are several methods known to the person skilled in the art, such as introduction of texturizing agents. Texturizing agents, such as stabilizers and gelatine can be used to reduce the amount of milk protein added. While the use of texturizing agents, such as stabilizers, in yogurt can be more cost effective than milk protein addition, their use is restricted by regulation and labelling laws.

Another solution for providing fermented milk products with increased texture is to add enzymes during the manufacturing process to proteolyse milk casein, such as kappa-casein. US2005/0095317 describes a kappa-caseinolytic treatment by using a chymosin, which is widely used in the production of cheese.

WO2015/128417 describes variants of chymosin with improved milk-clotting properties.

US2011/008492 describes methods for coagulating bovine milk and making a dairy product such as cheese using a coagulant identical or substantially identical to a chymosin originating from an animal of the Tylopoda suborder.

US2014/072670 describes methods of recombinantly producing non-bovine chymosin.

However, a drawback of adding proteolytic enzymes to the fermented product manufacturing process it that the proteolytic force in the derived fermented product, such as yogurt, is too high, which results in an increased ripening of the fermented product due to ongoing proteolysis during shelf life of the fermented product.

Therefore, there is a need in the art for a manufacturing process for fermented milk products, wherein the provided fermented milk product i.e., the yogurt, has a desired texture as well as a reduced ripening.

### Definitions

The term "**milk**" is intended to encompass milks from mammals and plant sources or mixtures thereof. Preferably, the milk is from a mammal source. Mammal sources of milk include, but are not limited to cow, sheep, goat, buffalo, camel, llama, mare and deer. In an embodiment, the milk is from a mammal selected from the group consisting of cow, sheep, goat, buffalo, camel, llama, mare and deer, and combinations thereof. Plant sources of milk include, but are not limited to, milk extracted from soy bean, pea, peanut, barley, rice, oat, quinoa, almond, cashew, coconut, hazelnut, hemp, sesame seed and sunflower seed. In addition, the term "milk" refers to not only whole milk, but also skim milk or any liquid component derived thereof.

As used in the present specification, the term "**fermented milk product**" refers to a product that has been fermented with lactic acid bacteria such as *Streptococcus thermophilus* and optionally *Lactobacillus delbruekii* subsp. *bulgaricus,* but also, optionally, other microorganisms such as *Lactobacillus delbruekii* subsp. *lactis, Bifidobacterium animalis* subsp. *lactis, Lactococcus lactis, Lactobacillus acidophilus* and *Lactobacillus casei,* or any microorganism derived therefrom. The lactic acid strains other than *Streptococcus thermophilus* and *Lactobacillus delbruekii* subsp. *bulgaricus,* are intended to give the finished product various properties, such as the property of promoting the equilibrium of the flora. The fermentation process increases the shelf-life of the product while enhancing and improving the digestibility of milk. Many different types of fermented milk products can be found in the world today. Examples are soured milk (e.g. buttermilk), soured cream and yogurt.

As used herein, the term "**yogurt**" is a fermented milk product produced by fermentation of milk by lactic acid bacteria, also known as "yogurt cultures". The fermentation of the lactose in the milk produces lactic acid which acts on the milk protein to give the yogurt its texture. Yogurt may be made from cow milk, the protein of which mainly comprises casein, which is most commonly used to make yogurt, but milk from sheep, goat, buffalo, camel, llama, mare, deer, water buffalo, ewes and/or mares, and combinations thereof may be used as well. The term "yogurt" furthermore encompasses, but is not limited to, yogurt as defined according to French and European regulations, e.g. coagulated dairy products obtained by lactic acid fermentation by means of specific thermophilic lactic acid bacteria only (i.e. *Lactobacillus delbruekii* subsp. *bulgaricus* and *Streptococcus thermophilus*) which are cultured simultaneously and are found to be living in the final product in an amount of at least 10 million CFU (colony-forming unit) per gram of the yogurt. Preferably, the yogurt is not heat-treated after fermentation. Yogurts may optionally contain added dairy raw materials (e.g. cream and/or protein) or other ingredients such as sugar or sweetening agents, one or more flavouring(s), cereals or nutritional substances, especially vitamins, minerals and fibers. Such yogurt advantageously meets the specifications for fermented milks and yogurts of the AFNOR NF 04-600 standard and/or the codex StanA-Ila-1975 standard. In order to satisfy the AFNOR NF 04-600 standard, the product must not have been heated after fermentation and the dairy raw materials must represent a minimum of 70 wt% of the finished product. Yogurt encompasses set yogurt, stirred yogurt, drinking yogurt, Petit Suisse, heat treated yogurt and yogurt-like products. Preferably, the yogurt is a stirred yogurt or a drinking yogurt. More preferably, the yogurt is a stirred yogurt.

The term "**starter culture composition**" or "**composition**" (also referred to as "**starter**" or "**starter culture**") as used herein refers to a composition comprising one or more lactic acid bacteria, which are responsible for the acidification of the milk base. Starter cultures compositions may be fresh (liquid), frozen or freeze-dried. Freeze dried cultures need to be regenerated before use. For the production of a fermented dairy product, the starter cultures composition is usually added in an amount from 0.01 to 3%, preferably from 0.01 and 0.02 % by weight of the total amount of milk base.

As used herein, the term "**lactic acid bacteria**" (LAB) or "**lactic bacteria**" refers to food-grade bacteria producing lactic acid as the major metabolic end-product of carbohydrate fermentation. These bacteria are related by their common metabolic and physiological characteristics and are usually Gram positive, low-GC, acid tolerant, nonsporulating, non-respiring, rod-shaped bacilli or cocci. During the fermentation stage, the consumption of lactose by these bacteria causes the formation of lactic acid, reduces the pH and leads to the formation of a (milk) protein coagulum. These bacteria are thus responsible for the acidification of milk and for the texture of the fermented milk product. As used herein, the term "lactic acid bacteria" or "lactic bacteria" encompasses, but is not limited to, bacteria belonging to the genus of *Lactobacillus* spp., *Bifidobacterium* spp., *Streptococcus* spp., *Lactococcus* spp., such as *Lactobacillus delbruekii* subsp. *bulgaricus, Streptococcus thermophilus, Lactobacillus lactis, Bifidobacterium animalis, Lactococcus lactis, Lactobacillus casei, Lactobacillus plantarum, Lactobacillus helveticus, Lactobacillus acidophilus* and *Bifidobacterium breve.*

Herein, **"chymosin"** typically indicates an aspartic protease, group 3.4.23.4 according to the Enzyme Nomenclature, 1992 of the International Union of Biochemistry and Molecular Biology, IUBMB. Chymosin is naturally produced by gastric chief cells in juvenile mammals. Chymosin is the main enzymatic component in rennet. Calf rennet is obtained of the lining of the abomasum (the fourth and final, chamber of the stomach) of young, unweaned calves.

Herein, the term **"C/P ratio"** refers to the clotting activity divided by the proteolytic activity of a specific enzyme sample. The method to measure the clotting activity (C) will quantify the efficiency of the enzyme sample to hydrolyse kappa-casein (k-CN) at a specific position (F105M106). The proteolytic activity (P) will quantify the ability of the coagulant to hydrolyse casein into small (TCA-soluble) peptide fragments and amino acids.

Herein, the term **"IMCU"** is understood International Milk Clotting Units. One IMCU equals about 0.126 nmol of bovine chymosin B (e.g. Maxiren or CHY-MAX). The strength of a milk clotting enzyme (such as chymosin enzyme present in a composition of the present invention) is determined as the milk clotting activity (IMCU per ml or per gram). Following the addition of diluted coagulant to a standard milk substrate, the milk will flocculate. The milk clotting time is the time period from addition of the coagulant until formation of visible flocks or flakes in the milk substrate. The strength of a coagulant sample is found by comparing the milk clotting time for the sample to that of a reference standard, a normal. This is expressed in IDF standard 157A:1997 which gives the IMCU definition: The total milk-clotting activity of the first batch of calf chymosin reference standard powder has once and for all been set at 1000 International Milk-Clotting Units per gram (IMCU/g). Further preparations of reference standards will be set relative to the previous reference. IMCU principle: Determination of the time needed for visible flocculation of renneted standard milk substrate with 0.05% calcium chloride, pH6.5. IMCU/ml of a sample is determined by comparison of the clotting time to that of a standard having known milk clotting activity and having the same enzyme composition of the sample.

Herein, the term **"ripening"** or alternatively maturation, is defined as maturing of the fermented milk product characterized by ongoing proteolysis during shelf life of the fermented product.

### Brief description of the sequence listing

SEQ ID NO: 1 sets out the nucleic acid sequence of the wild type pro-chymosin B gene sequence from *Bos taurus* with codon adaptation for expression in *K. lactis* and with linkers to allow cloning into pKLAC1.
SEQ ID NO: 2 sets out the amino acid sequence of the mature chymosin B sequence from *Bos taurus.*
SEQ ID NO: 3 sets out the nucleic acid sequence of the pro-chymosin sequence from *Camelus dromedarius* with codon adaptation for expression in *K. lactis.*
SEQ ID NO: 4 sets out the amino acid sequence of the mature chymosin sequence from *Camelus dromedarius.*
SEQ ID NO: 5 sets out the amino acid sequence of alpha-s1 casein from *Bos Taurus.*

### Detailed description of the invention

The present invention relates to a process for the production of yogurt, comprising fermenting milk with lactic acid bacteria and contacting the milk with a polypeptide having chymosin activity having a C/P ratio higher than the C/P ratio of bovine chymosin and wherein the polypeptide having chymosin activity has an amino acid sequence which;
a. when aligned with the chymosin comprising the sequence set out in SEQ ID NO: 2, comprises at least one substitution of an amino acid residue corresponding to amino acid 51 and/or 221 and wherein the polypeptide having chymosin activity shares at least about 90% sequence identity with a polypeptide according to SEQ ID NO: 2; and/or
**b.** when aligned with SEQ ID NO: 4 comprises at least one substitution of an amino acid residue located in the S2 binding pocket, and wherein an amino acid residue located in the S2 binding pocket corresponds to position 219, 223, 288, 290, 295 or 297 when aligned with SEQ ID NO: 4, and wherein the polypeptide having chymosin activity shares at least about 90% sequence identity with a polypeptide according to SEQ ID NO: 4.

The inventors of the present invention found that using a polypeptide having chymosin activity having a C/P ratio higher than the C/P ratio of bovine chymosin in the production of fermented milk products results in fermented milk products, such as yogurt, having a reduced ripening.

In a preferred embodiment, the present the polypeptide having chymosin activity is capable of hydrolysing bovine alpha s1-casein at position F23F24 so as to form αs1-I CN (f24-199) more rapidly than camel chymosin.

In another preferred embodiment, the C/P ratio is 2, for example 5, for example 10, such as 10 times higher than the C/P ratio of bovine chymosin. A polypeptide having chymosin activity of the invention may typically have a high specific milk clotting activity (C) and a low general, i. e. non-specific, proteolytic activity (P) with regard to milk proteins. Accordingly, the C/P ratio should preferably be as high as possible, as a relatively high P-value, during the yogurt manufacturing process and during shelf life of the yogurt will lead to the formation of low molecular peptides and free amino acids, which in turn may confer to the yogurt an undesirable bitter taste. C/P ratio may be expressed as a relative C/P ratio, for example in relation to a chymosin such as the bovine chymosin of SEQ ID NO: 2.

The present polypeptide having chymosin activity has an amino acid sequence which;
a. when aligned with the chymosin comprising the sequence set out in SEQ ID NO: 2, comprises at least one substitution of an amino acid residue corresponding to amino acid 51 and/or 221; and/or
b. when aligned with SEQ ID NO: 4 comprises at least one substitution of an amino acid residue located in the S2 binding pocket, for example at a position corresponding to amino acid 223.

Such a protein will typically be: capable of hydrolysing bovine alpha s1-casein at position F23F24 so as to form αs1-I CN (f24-199) more rapidly than camel chymosin; and have a C/P ratio higher than the C/P ratio of bovine chymosin.

Thus, at a position corresponding to amino acid 51 and/or 221 as defined with reference to SEQ ID NO: 2, a different amino acid may be present than is present at amino acid 51 and/or 221 in SEQ ID NO: 2. Thus, at a position corresponding to an amino acid in the S2 binding pocket as defined with reference to SEQ ID NO: 4, a different amino acid may be present than is present at that position within the S2 binding pocket in SEQ ID NO: 4.

A polypeptide having chymosin activity in the invention is a polypeptide having at least about 90% homology with SEQ ID NO: 2 or SEQ ID NO: 4, for example at least about 95%, at least about 98% or at least about 99% homology with SEQ ID NO: 2 or SEQ ID NO: 4.

Increased early development of fermented milk is related to the affinity of a specific region of alpha s1-casein to the different binding pockets in the peptide-binding groove of the coagulant. Accordingly, herein are described amino acids in the S2 binding pocket (Schechter en Berger (1967) Biochem. Biophys. Res. Commun. 27, 157-162) relevant for this affinity and this affinity may be modulated by altering amino acids in this pocket in order to increase the rate of hydrolysis of bovine alpha s1-casein at position F23F24 so as to form αs1-I CN (f24-199).

For example, introduction of a different amino acid side chain at position V223, as defined with reference to the sequence of the bovine chymosin, leads to a reduced first cut in alpha s1-casein. However, any chymosin with an amino acid change at the corresponding position, and other neighboring positions in the S2 binding pocket may lead to an altered affinity of the alpha s1-casein for the chymosin and a change in the kinetics of the first cut in alpha s1-casein.

Amino acid changes at the positions that are part of the S2 binding pocket are, most notably, T219, F223, Q288, D290, L295 and I297 in camel chymosin or amino acids at equivalent positions in other mammalian chymosins (e.g. T219, V223, Q288, E290, K295 and I297 in bovine chymosin), may also modulate the affinity of the alpha s1-casein for the chymosin, and thus alter the kinetics of the first cut in alpha s1 casein.

Accordingly, preferred polypeptides having chymosin activity of the invention may comprise the sequence set out in SEQ ID NO: 4 carrying one of the following mutations:
F223C, F223D, F223E, F223L, F223M, F223N, F223Q, F223V, F223Y, F223I Q288G, Q288H, Q288N, Q288R, Q288S
D290A, D290G, D290L, D290M, D290Q, D290S, D290T
L295F, L295I, L295K, L295M, L295R, L295T, L295Y, L295W
I297T, I297V

Combinations of such mutations at different positions may be used.

We describe that changes in amino acids A51 and K221 within the bovine chymosin amino acid sequence are important for an increased C/P. Introduction of these changes in a coagulant of choice will lead to a higher C/P value and therefore increased storage stability of the made yogurt.

Other positions that may be substituted (as defined with reference to the bovine sequence of SEQ ID NO: 2) are 48, 50, 61, 62, 109, 117, 126, 135, 144, 160, 161, 201, 202, 203, 221, 240, 242, 244, 254, 267, 280, 292 or 295. One or more of these positions may be changed so that it is different from the amino acid at that position as defined with reference to the bovine sequence of SEQ ID NO: 2.

Preferred chymosin polypeptides of the invention may comprise the sequence set out in SEQ ID NO: 2 carrying one of the following mutations or combinations of mutations:
A51V;
K221L;
K221M;
K221V;
V223Q;
A51V and K221V;
A51V and K221M;
A51V, K221V, S135T, A126G, S273Y and Q240E;
A51I and K221T; or
A51V, K221V, N50D, N144H, N160D, S201D, Q242E, M267E and Q280E

According to the invention, two properties are combined that permits, for example, the construction of a calf chymosin variant having higher C/P value useful or the construction of a camel chymosin variant having capable of more rapidly hydrolysing bovine alpha s1-casein at position F23F24 so as to form αs1-I CN.

Preferably, preferred polypeptides having chymosin activity of the invention are derived from a mammal, more preferably from a cow or from a camel.

Fermenting milk with lactic acid bacteria and contacting the milk with a polypeptide having chymosin activity having a C/P ratio higher than the C/P ratio of bovine chymosin may be carried at the same time. Alternatively, the milk is first contacted with the polypeptide having chymosin activity, and thereafter fermented with lactic acid bacteria. Vice versa, the milk is first fermented with lactic acid bacteria, and thereafter contacted with the polypeptide having chymosin activity. Alternatively, a part of the milk is contacted with a polypeptide having chymosin activity having a C/P ratio higher than the C/P ratio of bovine chymosin while another part of the milk is fermented with lactic acid bacteria, where after both parts can be combined to provide the fermented milk product i.e., the yogurt.

Preferably the present lactic acid bacteria selected from the group consisting of *Lactobacillus* spp., *Bifidobacterium* spp., *Streptococcus* spp., *Lactococcus* spp., such as *Lactobacillus delbruekii* subsp. *bulgaricus, Streptococcus thermophilus or Streptococcus thermophilus, Lactobacillus lactis, Bifidobacterium animalis, Lactococcus lactis, Lactobacillus casei, Lactobacillus plantarum, Lactobacillus helveticus, Lactobacillus acidophilus* and *Bifidobacterium breve.* More preferably the present lactic acid bacteria comprise *Streptococcus thermophilus* and *Lactobacillus delbruekii* subsp. *Bulgaricus.* This combination of strains is advantageous in for instance a process for the production of a fermented milk product such as yogurt or in the final properties of the fermented milk product such as yogurt. These strains may for instance further improve the acidification speed or they may confer certain flavours.

*Lactobacillus delbrueckii* ssp. *bulgaricus,* when present in the method of the invention, may constitute between 0.1% and 10% of the total cfu's of the present lactic acid bacteria, preferably between 0,2% and 5%, more preferably between 0,5% and 2%, more preferably between 0,8 and 1,2%, most preferably 1%.

In one embodiment, the process of the invention provides a yogurt wherein one or more textural attributes selected from the group consisting of rheology, appearance, the structure, mouthfeel, the after feel of the yogurt, has been improved. Most preferably, the invention provides a process wherein the rheology attributes of the yogurt, more preferably the Brookfield and/or the shear stress has been improved. In another preferred embodiment, the invention provides a process wherein the appearance of the yogurt, more preferably the shininess and/or the whiteness has been improved. In yet another preferred embodiment, the invention provides a process wherein the structure of the yogurt, more preferably the visual aspects such as ropiness and/or visual thickness and/or smoothness has been improved. In another preferred embodiment, the invention provides a process wherein the mouthfeel of the yogurt, more preferably the thickness and/or the creaminess and/or the sliminess and/or the melting and/or the astringency has been improved. In another preferred embodiment, the invention provides a process wherein the after feel of the yogurt, more preferably the astringency and/or the pungency and/or the fat coating has been improved.

Highly preferably, the invention provides a process of the invention wherein two or more, preferably three or more, more preferably four or more textural attributes selected from the group consisting of Brookfield and the shear stress and the shininess and the whiteness and ropiness and visual thickness and smoothness and the thickness and the creaminess and the astringency and the pungency and the fat coating has been improved.

The most preferred fermented milk product that is produced by the process of the invention is yogurt as defined hereinbefore.

The milk that may be used in the process of the invention, may be any milk suitable for the production of a fermented milk product, such as yogurt. Milk has been defined hereinbefore and may encompass milks from mammals and plant sources or mixtures thereof. Preferably, the milk is from a mammal source. Mammal sources of milk include, but are not limited to cow, sheep, goat, buffalo, camel, llama, mare and deer. In an embodiment, the milk is from a mammal selected from the group consisting of cow, sheep, goat, buffalo, camel, llama, mare and deer, and combinations thereof. Plant sources of milk include, but are not limited to, milk extracted from soy bean, pea, peanut, barley, rice, oat, quinoa, almond, cashew, coconut, hazelnut, hemp, sesame seed and sunflower seed. In addition, the term "milk" refers to not only whole milk, but also skim milk or any liquid component derived thereof. The fat content in the milk and in the subsequent fermented milk product i.e., the yogurt, may be as is known in the prior and as is referred in the background of the invention.

In a preferred embodiment, the present process comprising a step of inactivating the polypeptide having chymosin activity having a C/P ratio higher than the C/P ratio of bovine chymosin. For example inactivating by heat treatment.

According to another aspect, the present invention relates to yogurt, preferably yogurt having a reduced ripening or having a reduced proteolysis, comprising lactic acid bacteria and comprising a polypeptide having chymosin activity having a C/P ratio higher than the C/P ratio of bovine chymosin as defined before. Preferably, the present yogurt has a reduced ripening in comparison with a similar yogurt which comprises bovine chymosin. More preferably, the present yogurt has an increased texture shelf stability in comparison with a similar yogurt which comprises bovine chymosin.

According to another aspect, the present invention relates to a kit of parts comprising lactic acid bacteria and comprising a polypeptide having chymosin activity having a C/P ratio higher than the C/P ratio of bovine chymosin as defined before.

According to yet another aspect, the present invention relates to the use of the present polypeptide having chymosin activity having a C/P ratio higher than the C/P ratio of bovine chymosin as defined before producing yogurt, preferably for reducing the ripening in yogurt or for increasing the shelf life of yogurt such as for increasing the texture shelf stability of yogurt. More preferably the present invention relates to the use of the present polypeptide for producing a yogurt having a shelf life of more than 4, more than 5, or more than 6 weeks. Preferably more than 4, more than 5, or more than 6 weeks when stored under standard conditions in a refrigerator. Alternatively, the present invention relates to the use of the present polypeptide for increasing the shelf life of yogurt with at least 1, 2, 3, 4 or 5 weeks. More specifically, the present invention relates to the use of the present polypeptide for increasing the shelf life of yogurt with at least 1, 2, 3, 4 or 5 weeks in comparison with yogurt prepared with cow chymosin, preferably cow chymosin according to the amino acid sequence as shown in SEQ ID No 2.

### MATERIALS AND METHODS

### 1. Bacterial strains.

Starter culture Delvo^{®}Yog for stirred yogurt was used as direct inoculation starter, obtained from DSM Food Specialties B.V.

### 2. Chymosin.

Maxiren^{®} calf chymosin obtained from DSM Food Specialties B.V, and the polypeptides of the invention made as disclosed in WO2013164481.

### 3. Yogurt preparation

Stirred yogurt was made using a flow pasteurizer and a smoothener with back cooling. The milk composition was 3.4% fat and 3.5% protein and the starter culture was used. The rennets were added alongside the starter culture with a dosage of 0.0002% based on a formulation of 180 IMCU/mls. The fermentation temperature was 42°C. The acidification was monitored using Cinac at the same temperature. Once a pH of 4.6 was reached, the yogurts were smoothened and filled out in suitable containers. The containers were then stored at 4°C.

### 4. Sensory analysis of yogurts

The sensory panel consisted of 8 members who had a specific training in sensory evaluation of yogurts. Products were presented in 3-digit coded, white plastic isothermal cups stored at 4°C. The samples were approximately 10°C when they were tested. Panellists were provided with mineral water and plain crackers for palate cleaning between samples. The sessions were carried out in a temperature controlled room at 20°C under white lighting in individual booths.

Data acquisition was assisted by FIZZ Sensory Analysis Software. Both monadic and hedonic scales are being used to rate the flavour and the texture attributes of products. The attributes were evaluated in the following order: visual texture with a spoon, texture-in-mouth, taste and aroma.

### 5. Sensory analysis of yogurts QDA

Descriptive sensory analysis was done by using the Quantitative Descriptive Analysis Method (Stone, H. and Sidel, J.L. "Sensory Evaluation Practises" 3rd dition, 2004). First, the panellists developed a list of attributes including definitions by means of evaluating a wide variety of references and a wide array of yogurts. Secondly, training sessions were organized to enable panellists to learn to consistently differentiate and replicate the yogurt samples. During the actual QDA measurements the intensities of the selected attributes were obtained per product by the FIZZ (Biosystems; France) sensory data acquisition system, using unstructured line scales ranging from 0 - 100. The products were offered semi-monadically and evaluated twice by the panellists (n=14) by means of a Balanced Complete Block design to avoid sequence effects. Statistical analysis of the data was done by analysis of variance with Fisher's least significant difference (LSD) as a post hoc test (SenPaq) and modelled using Principle Component analysis (PCA) (SenPaq).

### 6. Shear stress of yogurt

The samples were measured using a Physica MCR501 rheometer equipped with a concentric cylinder measurement system (CC-27). A solvent trap was used to prevent evaporation of water as much as possible. The samples were slightly stirred with a spoon before loading into the rheometer. Before measurement the samples were allowed to rest and heat/cool to the measuring temperature (25°C) for 5 minutes. A standard experimental protocol was applied consisting out of the following two measuring sequences:
1. A strain sweep to determine the initial gel strength (dynamic shear modulus): this is an oscillatory test where at a fixed angular frequency (omega = 10 rad/s) an increasing amplitude is applied: on a logarithmic scale the amplitude is increased from 0.01 to 100 % with 5 measuring points per decade.
2. After the strain sweep the yogurts are allowed to rest for 30 seconds in the rheometer and subsequently a shear rate sweep is applied to determine the shear stress in mouth: This consists of applying an increasing shear rate to the yogurts ranging from 0.001 to 1000 s⁻¹ on a logarithmic scale with 3 measuring points per decade (no fixed time setting: the rheometer software determines the required shearing time per measuring point).

This experiment gives a flow curve whereby the measured stress is plotted as a function of the applied shear rate. This curve can then be combined with literature data to determine the relevant shear stress in the mouth as explained in the following.

By sensory panelling of various food products Shama and Sherman identified windows of instrumental shear stresses and shear rates corresponding to products with similar thickness ratings but different shear-thinning behavior. These windows correspond to the rheological regimes applied in the mouth during thickness rating. The governing shear rate was shown to be dependent on the viscosity of the product itself. (see Figure 1 from Shama, F. and Sherman, P. Journal of Texture Studies, 4, 111-118. (1973), "Identification of stimuli controlling the sensory evaluation of viscosity II oral methods").

For the yogurts of the example below the (predicted) shear stress in the mouth is determined by plotting the experimentally measured flow curves (measured shear stress in function of applied shear rate of the shear rate sweep experiment described above) onto the aforementioned Figure 1 from Shama and Sherman. The predicted shear stress in the mouth is defined as the cross-over between the measured flow curves and the upper bound of the "shear rate - shear stress" windows of Figure 1 of Shama and Sherman. In figure 2 the authors give examples for various food stuffs. The thus derived shear stress gave a good correlation with the sensory perception of thickness in the mouth.

### 7. Brookfield

Viscosity measurements were performed using a Brookfield RVDVII+ Viscometer, which allows viscosity measurement on an undisturbed product (directly in the pot). The Brookfield Viscometer determines viscosity by measuring the force required to turn the spindle into the product at a given rate. The Helipath system with a T-C spindle was used as it is designed for non-flowing thixotropic material (gels, cream). It slowly lowers or raises a rotating T-bar spindle into the sample so that not always the same region of the sample is sheared (helical path). Thus, the viscometer measures constantly the viscosity in fresh material and is thus considered to be the most suitable for measuring stirred yogurt viscosity. A speed of 30 rpm was used for 31 measuring points, at an interval of 3 seconds. The averages of the values between 60 and 90 seconds are reported.

### EXAMPLES

### Example 1

Yogurt was prepared as in the materials and methods with a polypeptide of the invention and the results are shown in table 1 below.

| | **Maxiren^{®}** | **Polypeptide of the invention** |
|---|---|---|
| **Predicted shear stress in the mouth (Pa)** | 18.95 | 19.08 |
| **Quality of yogurt after 4 weeks** | Good | Good |
| **Quality of yogurt after 5 weeks** | Overripe | Good |
| **Quality of yogurt after 6 weeks** | Overripe | Good |

The above results show that the predicted shears stress is more or less comparable, whereas the yogurt produced with the present polypeptide provides a reduced ripening of the yogurt.

## Claims

1. A process for the production of yogurt, comprising fermenting milk with lactic acid bacteria and contacting milk with a polypeptide having chymosin activity having a **clotting activity divided by proteolytic activity** (C/P ratio) higher than the C/P ratio of bovine chymosin and wherein the polypeptide having chymosin activity has an amino acid sequence which;
a. when aligned with the chymosin comprising the sequence set out in SEQ ID NO: 2, comprises at least one substitution of an amino acid residue corresponding to amino acid 51 and/or 221 and wherein the polypeptide having chymosin activity shares at least about 90% sequence identity with a polypeptide according to SEQ ID NO: 2; and/or
b. when aligned with SEQ ID NO: 4 comprises at least one substitution of an amino acid residue located in the S2 binding pocket, and wherein an amino acid residue located in the S2 binding pocket corresponds to position 219, 223, 288, 290, 295 or 297 when aligned with SEQ ID NO: 4, and wherein the polypeptide having chymosin activity shares at least about 90% sequence identity with a polypeptide according to SEQ ID NO: 4.

2. Process according to claim 1, wherein the C/P ratio is 2, for example 5, for example 10, such as 10 times higher than the C/P ratio of bovine chymosin.

3. Process according to any one of claims 1 to 2, wherein the polypeptide having chymosin activity comprises the sequence set out in SEQ ID NO: 2 carrying one of the following mutations or combinations of mutations:
A51V;
K221L;
K221M;
K221V;
A51V and K221V;
A51V and K221M;
A51V, K221V, S135T, A126G, S273Y and Q240E;
A51I and K221T; or
A51V, K221V, N50D, N144H, N160D, S201D, Q242E, M267E and Q280E.

4. A process according to any one of claims 1 to 62, wherein the polypeptide having chymosin activity comprises the sequence set out in SEQ ID NO: 4 carrying one of the following mutations: F223C, F223D, F223E, F223L, F223M, F223N, F223Q, F223V, F223Y, F223I, Q288G, Q288H, Q288N, Q288R, Q288S, D290A, D290G, D290L, D290M, D290Q, D290S, D290T, L295F, L295I, L295K, L295M, L295R, L295T, L295Y, L295W, I297T or I297V.

5. Process according to any of the preceding claims, wherein the polypeptide having chymosin activity is derived from a mammal.

6. Process according to any of the claims 1 to 3 or 5, wherein the polypeptide having chymosin activity is derived from a cow.

7. Process according to any of the claims 1 to 2, 4 or 5, wherein the polypeptide having chymosin activity is derived from a camel.

8. Yogurt having a reduced ripening, comprising lactic acid bacteria and a polypeptide having chymosin activity as defined in any of the claims 1 to 7.

9. A kit of parts comprising lactic acid bacteria and a polypeptide having chymosin activity as defined in any of the claims 1 to 7, and wherein said lactic acid bacteria comprise *Streptococcus thermophilus* and *Lactobacillus delbruekii* subsp. *bulgaricus.*

10. Use of a polypeptide having chymosin activity as defined in any of the claims 1 to 7 for the production of yogurt.

11. Use of a polypeptide having chymosin activity as defined in any of the claims 1 to 7 for reducing the ripening in yogurt or for increasing the shelf life of yogurt, compared to a yogurt which comprises bovine chymosin.

## Patentansprüche

1. Verfahren zur Herstellung von Joghurt, umfassend Fermentieren von Milch mit Milchsäurebakterien und Inkontaktbringen von Milch mit einem Polypeptid mit Chymosinaktivität, das eine Gerinnungsaktivität geteilt durch proteolytische Aktivität (C/P-Verhältnis) aufweist, die größer als das C/P-Verhältnis von Rinder-Chymosin ist, und wobei das Polypeptid mit Chymosinaktivität eine Aminosäuresequenz aufweist, die; a bei Ausrichtung am Chymosin, das die Sequenz unter SEQ ID NO: 2 umfasst, mindestens eine Substitution eines Aminosäurerestes, der Aminosäure 51 und/oder 221 entspricht, umfasst, und wobei das Polypeptid mit Chymosinaktivität mindestens etwa 90 % Sequenzidentität mit einem Polypeptid gemäß SEQ ID NO: 2 teilt; und/oder b. bei Ausrichtung an SEQ ID NO: 4 mindestens eine Substitution eines Aminosäurerestes, der sich in der S2-Bindungstasche befindet, umfasst, und wobei ein Aminosäurerest, der sich in der S2-Bindungstasche befindet, Position 219, 223, 288, 290, 295 oder 297 bei Ausrichtung an SEQ ID NO: 4 entspricht und wobei das Polypeptid mit Chymosinaktivität mindestens etwa 90 % Sequenzidentität mit einem Polypeptid gemäß SEQ ID NO: 4 teilt.

2. Verfahren nach Anspruch 1, wobei das C/P-Verhältnis 2-, zum Beispiel 5-, zum Beispiel 10-, wie 10-mal größer ist als das C/P-Verhältnis von Rinder-Chymosin.

3. Verfahren nach einem der Ansprüche 1 bis 2, wobei das Polypeptid mit Chymosinaktivität die Sequenz unter SEQ ID NO: 2 umfasst, die eine der folgenden Mutationen oder Kombinationen von Mutationen trägt:
A51V;
K221L;
K221M;
K221V;
A51V und K221V;
A51V und K221M;
A51V, K221V, S135T, A126G, S273Y und Q240E;
A51I und K221T; oder
A51V, K221V, N50D, N144H, N160D, S201D, Q242E, M267E und Q280E.

4. Verfahren nach einem der Ansprüche 1 bis 2, wobei das Polypeptid mit Chymosinaktivität die Sequenz unter SEQ ID NO: 4 umfasst, die eine der folgenden Mutationen trägt: F223C, F223D, F223E, F223L, F223M, F223N, F223Q, F223V, F223Y, F2231, Q288G, Q288H, Q288N, Q288R, Q288S, D290A, D290G, D290L, D290M, D290Q, D290S, D290T, L295F, L2951, L295K, L295M, L295R, L295T, L295Y, L295W, I297T oder I297V.

5. Verfahren nach einem der vorhergehenden Ansprüche, wobei das Polypeptid mit Chymosinaktivität von einem Säugetier stammt.

6. Verfahren nach einem der Ansprüche 1 bis 3 oder 5, wobei das Polypeptid mit Chymosinaktivität von einer Kuh stammt.

7. Verfahren nach einem der Ansprüche 1 bis 2, 4 oder 5, wobei das Polypeptid mit Chymosinaktivität von einem Kamel stammt.

8. Joghurt mit reduzierter Reifung, umfassend Milchsäurebakterien und ein wie in einem der Ansprüche 1 bis 7 definiertes Polypeptid mit Chymosinaktivität.

9. Kit-of-parts, umfassend Milchsäurebakterien und ein wie in einem der Ansprüche 1 bis 7 definiertes Polypeptid mit Chymosinaktivität, und wobei die Milchsäurebakterien *Streptococcus thermophilus* und *Lactobacillus delbruekii* subsp. *bulgaricus* umfassen.

10. Verwendung eines wie in einem der Ansprüche 1 bis 7 definierten Polypeptids mit Chymosinaktivität zur Herstellung von Joghurt.

11. Verwendung eines wie in einem der Ansprüche 1 bis 7 definierten Polypeptids mit Chymosinaktivität zum Reduzieren der Reifung bei Joghurt oder Erhöhen der Haltbarkeit von Joghurt im Vergleich zu einem Joghurt, der Rinder-Chymosin umfasst.

## Revendications

1. Procédé pour la production de yaourt, comprenant la fermentation de lait avec des bactéries lactiques et la mise en contact du lait avec un polypeptide ayant une activité de chymosine ayant une activité de coagulation divisée par l'activité protéolytique (rapport C/P) supérieur au rapport C/P de la chymosine bovine et dans lequel le polypeptide ayant une activité de chymosine a une séquence d'acides aminés qui ;
a. lorsqu'il est aligné avec la chymosine comprenant la séquence présentée dans SEQ ID NO: 2, comprend au moins une substitution d'un résidu d'acide aminé correspondant à l'acide aminé 51 et/ou 221 et dans lequel le polypeptide ayant une activité de chymosine partage au moins environ 90 % d'identité de séquence avec un polypeptide selon SEQ ID NO: 2 ; et/ou
b. lorsqu'il est aligné avec SEQ ID NO : 4 comprend au moins une substitution d'un résidu d'acide aminé situé dans la poche de liaison S2, et dans lequel un résidu d'acide aminé situé dans la poche de liaison S2 correspond à la position 219, 223, 288, 290, 295 ou 297 lorsqu'il est aligné avec SEQ ID NO: 4, et dans lequel le polypeptide ayant une activité de chymosine partage au moins environ 90 % d'identité de séquence avec un polypeptide selon SEQ ID NO: 4.

2. Procédé selon la revendication 1, dans lequel le rapport C/P est 2, par exemple 5, par exemple 10, par exemple 10 fois plus élevé que le rapport C/P de la chymosine bovine.

3. Procédé selon l'une quelconque des revendications 1 à 2, dans lequel le polypeptide ayant une activité de chymosine comprend la séquence présentée dans SEQ ID NO: 2 portant l'une des mutations ou combinaisons de mutations suivantes :
A51V ;
K221L ;
K221M ;
K221V ;
A51V et K221V ;
A51V et K221M ;
A51V, K221V, S135T, A126G, S273Y ET Q240E ;
A51I et K221T ; ou
A51V, K221V, N50D, N144H, N160D, S201D, Q242E, M267E et Q280E.

4. Procédé selon l'une quelconque des revendications 1 à 62, dans lequel le polypeptide ayant une activité de chymosine comprend la séquence présentée dans SEQ ID NO : 4 portant l'une des mutations suivantes : F223C, F223D, F223E, F223L, F223M, F223N, F223Q, F223V, F223Y, F223I, Q288G, Q288H, Q288N, Q288R, Q288S, D290A, D290G, D290L, D290M, D290Q, D290S, D290T, L295F, L295I, L295K, L295M, L295R, L295T, L295Y, L295W, I297T ou I297V.

5. Procédé selon l'une quelconque des revendications précédentes, dans lequel le polypeptide ayant une activité de chymosine est dérivé d'un mammifère.

6. Procédé selon l'une quelconque des revendications 1 à 3 ou 5, dans lequel le polypeptide ayant une activité de chymosine est dérivé d'une vache.

7. Procédé selon l'une quelconque des revendications 1 à 2, 4 ou 5, dans lequel le polypeptide ayant une activité de chymosine est dérivé d'un chameau.

8. Yaourt ayant un mûrissement réduit, comprenant des bactéries lactiques et un polypeptide ayant une activité de chymosine tel que défini dans l'une quelconque des revendications 1 à 7.

9. Kit de pièces comprenant des bactéries lactiques et un polypeptide ayant une activité de chymosine tel que défini dans l'une quelconque des revendications 1 à 7, et dans lequel lesdites bactéries lactiques comprennent *Streptococcus thermophilus* et *Lactobacillus delbruekii* subsp. *bulgaricus.*

10. Utilisation d'un polypeptide ayant une activité de chymosine tel que défini dans l'une quelconque des revendications 1 à 7 pour la production de yaourt.

11. Utilisation d'un polypeptide ayant une activité de chymosine tel que défini dans l'une quelconque des revendications 1 à 7 pour réduire la maturation dans un yaourt ou pour augmenter la durée de conservation d'un yaourt, par comparaison avec un yaourt qui comprend de la chymosine bovine.
